# EUROPEAN PATENT APPLICATION

(11) **EP 3 318 250 A2**
(43) Date of publication of application: **09.05.2018**
(21) Application number: 16818323.4
(22) Date of filing: 30.06.2016
(51) Int. Cl.: A61K 31/00, A61K 31/4045, A61K 31/57, A61P 15/00

(54) **METHOD FOR CONTROLLING SEXUAL BEHAVIOUR AND CONTRACEPTION IN FEMALE MAMMALS**

(30) Priority: 01.07.2015 RU 2015126106
(71) Applicant: Obshchestvo S Ogranichennoj Otvetstvennost'yu "Nauchno-Vnedrencheskij Centr Agrovetzashchita", 129329 Moscow (RU)
(72) Inventor: ENGASHEV, Sergey Vladimirovich, Moscow 127221 (RU); GERMANOVA, Mariya Sergeevna, G. Obninsk 249032 (RU); GERMANOV, Sergey Borisovich, Novokuznetsk 654084 (RU); ENGASHEVA, Ekaterina Sergeevna, Moscow 127282 (RU); NOVIKOV, Denis Dmitrievich, G. Bolkhov 303142 (RU); GLADUSHKO, Yana Yur'evna, G. Lomonosov 188512 (RU); KHOMISHIN, Dmitriy Vladimirovich, Moskovskaya obl. 143333 (RU); GERMANOVA, Ol'ga Leonidovna, G. Novokuznetsk 654084 (RU)
(74) Representative: Lapienis, Juozas
(86) International application number: PCT/RU2016/000403
(87) International publication number: WO 2017/003321

(57) **Abstract**

The invention relates to the veterinary field in particular to a method for temporarily blocking sexual functions in female mammals, including behavioural phenomena (search for a partner, vocalization, etc.). The method for controlling sexual behaviour and contraception in female animals comprises administration of melatonin, gestagen and α-adrenergic blocking agent tropic to the posterior nuclei of the hypothalamus, while the combination of specified biologically active substances (BAS) is administered in therapeutic doses once during any period of the estrous cycle. A pharmaceutical composition for controlling sexual behaviour of female animals intended for the "Spot-on" transdermal administration, having pH in the range of 4.0 to 6.0 and containing therapeutic quantities of melatonin, gestagen and α-adrenergic blocking agent tropic to the posterior nuclei of the hypothalamus with the following ratio of the components (mass%): gestagen - therapeutic quantities; melatonin - therapeutic quantities; α-adrenergic blocking agent tropic to the posterior nuclei of the hypothalamus - therapeutic quantities; N-methyl-N-(1,2*R*,3*R*,4*R*,5*S*-pentahydroxyhexyl)-octadecanamide - 0.5; *D*-panthenol - 0.5; polyoxyethylated α-tocopherol - 10; polyoxyethylated lanolin - 1; 3-(octadecyloxy)-1,2-propanediol - 0.1; carboxylic acid - 0.1-1.1; N,N-diethyltoluamid - 15; 1,2-isopropylidene glycerol - 20; 1,2,3-triacetoxypropane - up to 100. The invention makes it possible, while using the minimal dose of the steroid hormone harmful to health of animals, to inhibit sexual behaviour of female animals for the period of 2 months to half a year (depending on the length of the daytime period). The pharmaceutical composition is intended for transdermal administration. The pharmaceutical composition also can be used in humane medicine for effective and safe contraception in the form of metered dose transdermal sprays for convenient application.

## Description

### Field of the Invention

The invention relates to the veterinary field in particular to a method for temporarily blocking sexual functions in female mammals, including behavioural phenomena (search for a partner, vocalization, etc.). The method involves single/double administering to an animal of pharmaceutical composition comprising α-adrenergic blocking agent tropic to the posterior nuclei of the hypothalamus, melatonin and steroid compound with gestagen activity as active substances. The method makes it possible, while using the minimal dose of the steroid hormone harmful to health of animals, to inhibit sexual behaviour of female animals for the period of 2 months to half a year (depending on the length of the daytime period). A pharmaceutical composition is intended for the transdermal administration. The pharmaceutical composition also can be used in humane medicine for effective and safe contraception in the form of metered dose transdermal sprays for convenient application.

### Background of the Invention

Pharmaceutical effects on sexual functions of female animals (mammals) in order to achieve both contraceptive effect and, to a greater extent, the inhibition of sexual behaviour (search for a partner, vocalization, restless behaviour, aggressiveness), is of great importance in veterinary medicine. It should be noted that the problems of pharmaceutical effects in the regulation of sexual functions in veterinary and humane medicine are different. When veterinary medicine is aimed to have a blocking effect on the sexual behaviour of animals uncomfortable for their owners with achievement of indirect protection from unwanted pregnancies; humane medicine is, on the contrary, aimed to effective contraception with as lower influence on the psychology of women as possible. In addition, oral administration of drugs to restless animals that often totally refuse food is a major problem in veterinary medicine. Forced oral administration of drugs often causes aggression, intense salivation and vomiting in animals. Therefore, the "multiphase" estrogen/gestagen combinations popular in humane medicine, which require daily administration according to a certain regimen for reliable contraception, are not applied in veterinary medicine. Surgical methods in particular sterilization are the most widely used in the veterinary medicine of small domestic animals, however this method has a number of disadvantages for females:
1. The major surgery in females is traumatic, complex and unsafe with frequent complications, including fatal outcomes; the period of convalescence is very long;
2. The impact on the animal's organism is irreversible, breeding in the future is impossible;
3. After the surgery, behaviour of the animal and metabolism in its organism change undesirably more or less. The animal becomes less active, play and cognitive activities decrease, tendency to obesity and to a number of concomitant diseases appear.

The reversible chemical castration of female mammals by the administration of hypothalamic releasing hormones (goserelin, buserelin, etc.), widely used in humane medicine in the treatment of hormone-dependent oncological diseases, is practically not used in veterinary medicine of small domestic animals due to complicated regimens of administration in the absence of convenient veterinary pharmaceutical forms and high price for the procedure which is much higher than the price for surgical castration.

Most commonly used regulators of the sexual functions of female animals in the veterinary medicine of small domestic animals are gestagens in particular those that are capable of direct and strong influence on the hypothalamic and pituitary structures, which leads to suppression of gonadotropins production and therefore to achievement both of contraception and certain impact on the behavioural phenomena of sexual behaviour of female animals. Progestagen compounds capable of preserving pregnancy in animals after ovariectomy and causing secretion of uterine glands were identified, isolated in pure form and eventually synthesized in the first half of the 20th century. In the 1930s it was found that intramuscular injections of progesterone are capable of blocking ovulation in rabbits. Screening performed among progesterone-like compounds capable of regulating the reproductive cycle led to the synthesis of a variety of new compounds which were expected to have contraceptive activity. There were medroxyprogesterone acetate (MPA), melengesterol acetate, megestrol acetate and many others among them. Many progesterone derivatives have been studied in small animals.

The most widely used compounds in North America, Europe and Australia/New Zealand include medroxyprogesterone acetate, megestrol acetate and proligestone. Commercial drugs based on such compounds as delmadinone acetate are less commonly used and less available in some European countries.

Pharmaceutical compositions based on megestrol acetate are the most widely used as contraceptive drugs for small domestic animals in the Russian veterinary pharmaceutics. Patent RU2163809 published on 10.03.2001 provides composition comprising micronized megestrol acetate with particle size of 2-30 µm and polyethyleneglycol-400; concentration of megestrol acetate in the mixture is from 20 to 90 g/l. Megestrol acetate was developed as a drug with a shorter action than MPA. Accelerated metabolism makes the drug more suitable for short-term, temporary estrus detainment and its suppression. The side effects of megestrol acetate on the part of the uterus, mammary glands and metabolism are similar to those of any other progestin.

Patent RU2233586, published on 10.08.2004 provides use of modified (synthetic) gestagen in combination with central anticholinergic drug or adrenergic blocking agent as antifertility (contraceptive agent) for animals; proroxan is provided as latter. However, the achieved effect is insignificant (contraception coefficient of 55%), the synthetic gestagen used is not available in the market and the drug is to be administered orally or by means of injections for a long time according to a complex regimen.

Detected comprehensive regulating action of melatonin on the sexual sphere is known in the prior art. Patents RU2067001, published on 27.09.1996, RU2114621 published on 10.07.1998, US4855305, published on 08.08.1989 provide use of combination of melatonin and gestagen or (and) estrogen for achievement of contraceptive effect. In this case the side effects of steroid hormones described above are minimized. However, provided drugs require complex administration regimens and frequently administration by injections, which is little acceptable for veterinary use.

The object of this invention is to find the most safe and convenient for use method for temporarily blocking sexual functions in female mammals, including behavioural phenomena (search for a partner, vocalization, etc.). Technical result of this invention consists in obtaining of high-efficiency pharmaceutical composition for "Spot-on" application during any period of estrous cycle, which helps to solve the problems of change in sexual behaviour of female mammals.

### Disclosure of the invention

When studying contraceptive effects of the combination of melatonin and progestagens with the purpose of obtaining at least suppression effect to aggressive reactions in animals in mating season, we have additionally introduced known non-selective α-adrenergic blocking agent, proroxan [M.D. Mashkovsky, "Drugs"/M., Novaya Volna, 2012], for which there is a description of specific accumulation in the posterior nuclei of the hypothalamus accompanied with suppression of aggressiveness and other favorable effects without any sedative phenomena and significant undesired effects. It was surprisingly found that in such case estrus is stopped in animals after one/two administrations of the above combination of biologically active substances and blocked for a period over 2 months at long daytime period (spring) and up to 6-8 months during impact on an animal at short daytime period (fall). Estrus suppression is also accompanied with simultaneous and prompt blocking of behavioural phenomena of sexual behaviour - search for a partner, vocalization, aggressiveness, rut, etc. The effect is developed in 2-4 hours which does not give evidence of contribution of the used gestagen and melatonin doses. The remarkable thing is that behaviour of animals acquires strongly pronounced juvenile characteristics: play and investigative behaviour is observed, and the animal becomes friendly and active.

Proroxan or(and) butyroxan, which are similar in properties, may be used as a non-selective α-adrenergic blocking agent; proroxan, however, causes greater and faster effect. Other approved selective and non-selective adrenergic blocking agents (see example 35) haven't resulted in the described effect.

Any of the following gestagens employed in practice may be used as a gestagenic component: megestrol acetate, or (and) progesterone, or (and) oxyprogesterone capronate, or (and) dydrogesterone, or (and) pregninum, or (and) norethisterone, or (and) levonorgestrel, or (and) allylestrenol, or (and) lynestrenol, or (and) gestodene, or (and) dienogest, or (and) drospirenone, or (and) gestonorone capronate, or (and) medroxyprogesterone, or (and) mepregenol acetate, or (and) mepregenol acetate butyrate, or (and) mepregenol acetate hemisuccinate. The only advantage of megestrol acetate is that it is well known in the veterinary medicine of small domestic animals.

For gestagen and α-adrenergic blocking agent the doses should not exceed the commonly used therapeutic ones, because otherwise the likelihood of adverse side effects increases significantly. Melatonin is practically safe at a very significant overdosing.

One might expect that melatonin and proroxan as a part of transdermal forms will have a good transcutaneous bioavailability, and gestagen, the amounts of which in the blood are not critical for the development of the desired effect, as in particular pharmaceutical composition, will get into the blood stream in a sufficient amount and have a systemic exposure at the lowest local effects. A pharmaceutical composition meant for transdermal administration has been selected, containing (mass%):

| | |
|---|---|
| Gestagen | Therapeutic quantities |
| Melatonin | Therapeutic quantities |
| Proroxan base or (and) butyroxan | Therapeutic quantities |
| N-Methyl-N-(1,2*R*,3*R*,4*R*,5*S*-pentahydrox yhexyl)octadecanamide (stearylamide meglumine) | 0.5 |
| *D*-Panthenol | 0.5 |
| Polyoxyethylated α-tocopherol | 10 |
| Polyoxyethylated lanolin | 1 |
| 3-(Octadecyloxy)-1,2-propanediol (Batilol) | 0.1 |
| Carboxylic acid | 0.1-1.1 |
| N,N-Diethyltoluamid | 15 |
| 1,2-Isopropylidene glycerol | 20 |
| 1,2,3-Triacetoxypropane | Up to 100 |

which provided for a biological effect at "Spot-on" administration similar to oral administration methods; use of this drug is, however, more convenient. No skin irritation is detected, animals are unaware of the administration process and do not feel any discomfort in the area of application, so there is no scratching or licking.

The best results have been observed with a combination of the following components.

| | |
|---|---|
| Megestrol acetate | 0.5-2 |
| Melatonin | 10-20 |
| Proroxan base | 1-2 |
| N-Methyl-N-(1,2*R*,3*R*,4*R*,5*S*-pentahy droxyhexyl)-octadecanamide | 0.5 |
| *D*-Panthenol | 0.5 |
| Polyoxyethylated α-tocopherol | 10 |
| Polyoxyethylated lanolin | 1 |
| 3-(Octadecyloxy)-1,2-propanediol | 0.1 |
| Stearic acid | 1.1 |
| N,N-Diethyltoluamid | 15 |
| 1,2-Isopropylidene glycerol | 20 |
| 1,2,3-Triacetoxypropane | Up to 100 |

Lower concentrations of biologically active substances are selected based on the minimum amounts of BAS, resulting in an assured effect with a technically admissible amount of the "Spot-on" pharmaceutical form for a given animal species. Thus the optimum volume for cats is 0.5-2.0 ml, as in case of application of a larger amount of "Spot-on" product it will spread over an intolerably large area and dampen the hair. The upper concentration limits for proroxan and megestrol acetate are selected based on the known toxicity data for these components at convenient amounts of "Spot-on" applied form and their solubility in the pharmaceutical composition. The upper concentration limit for melatonin is defined by its solubility in the selected solvent composition.

The necessity for pH stabilization at the level of 4-6 was also found important, as the higher pH means faster BAS destruction (for instance, megestrol acetate), and the lower pH means the likelihood of skin irritation in the animal (see examples 38 and 39). This pH value is easily obtained by adding corresponding carboxylic acid additive with ionometric control: glycyrrhizinic, or(and) succinic, or(and) lactic, or(and) pyruvic, or(and) citric, or(and) malic, or(and) tartaric, or (and) stearic, or (and) myristic, or (and) octanoic, or (and) lauric acids (see examples 23, 25, 31, 39 and other), etc. Practice has shown that the desired quantity of carboxylic acid varies depending on the molecular mass and dissociation degree from 0.1 to 1.1 mass%.

The conducted biological tests (examples 2, 3, 35) have shown that the developed pharmaceutical composition at "Spot-on" application during any period of estrous cycle of female animal efficiently prevents or blocks estrus and after 2-4 hours blocks behavioural phenomena of sexual behaviour, while in case of the long daytime period (spring) the effect remains for no less than 2 months without repeated application of the drug, and in case of the short daytime period (fall) it lasts for more than six months, which allows to minimize the amount of steroid hormone injected to the animal's organism and therefore to minimize the likelihood of grievous and often lethal side effects, including the growth of hormone-dependent tumors. Such effect is not accompanied by symptoms of false pregnancy which is typical for many contraception methods in veterinary medicine and which leads to serious health deviations in the animal requiring treatment. Juvenile characteristics - play and investigative behaviour, non-aggressiveness - observed after application of the drug, are beneficial for the owners of animals.

The developed pharmaceutical composition may be used in humane medicine for rapid, safe and efficient contraception; the drug is used in the form of metered dose transdermal spray for convenient application (example 31). Unlike any known contraceptive agents, this drug does not require its everyday use for pre-coital contraceptive effect, excludes the likelihood of uterine bleeding, common at application of high-dose oral post-coital gestagenic contraceptives, eliminates the possibility of thrombophlebitis development. The effect after a single application lasts for no less than two months. According to literature data [B.G. Katzung. Basic and Clinical Pharmocology. Volume 2 / M.: Binom, 2008; 784 pages], melatonin contained in the composition has a rejuvenating effect on female organism, enhances emotional background, has strong antioxidant and adaptogene effect, and normalizes sleep.

### Embodiments

### Example 1

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.31 by adding stearic acid with the control by an ion meter with input impedance of > 2 GOhm. The beaker is wrapped with nontransparent foil. 10 g of proroxan, 100 g of melatonin and 10.0 g of megestrol acetate are added to the obtained solution. The mass is being agitated when heated to 40°C till complete dissolution of the components. pH value is adjusted by adding stearic acid to the value of 4.34 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.13 g/cm³;
3. pH 4.34;
4. Content of megestrol acetate is 11.12 mg/cm³;
5. Content of melatonin is 112.78 mg/cm³;
6. Content of proroxan is 11.17 mg/cm³;
7. Viscosity - 4.6 cPs.

The drug is packed in polypropylene-polyvinyl chloride pipettes of 1 ml.

After two years of storage the parameters of the drug are as follows:
1. Description - moving cloudy liquid mass, light-yellow solution;
2. Density - 1.10 g/cm³;
3. pH 4.28;
4. Content of megestrol acetate is 10.51 mg/cm³;
5. Content of melatonin is 111.02 mg/cm³;
6. Content of proroxan is 10.67 mg/cm³;
7. Viscosity - 4.6 cPs.

Thus the drug is stable when stored under natural conditions in polymer pipettes for 2 years.

### Example 2 (Effects: regulating sexual behaviour)

Testing of the drug obtained in accordance with example 1, by definition regulating sexual behaviour of animals, was carried out for apparently healthy fertile female cats (at the age of 10 months to 11 years with the mass of 1.5 to 4.0 kg) and dogs (at the age of 10 months to 10 years with the mass of 4 to 27 kg).

The cats/dogs were divided into two groups: control and experimental.

The drug was used once during the evening time of the day by application to dry intact skin in the area of back between the scapulas at the following dose: 1 dropper pipette (1 ml) per animal with the mass of less than 5 kg, 2 dropper pipettes - for animals with the mass of 5 to 15 kg, and 4 dropper pipettes - for animals with the mass of 15-35 kg. In case the desired effect is not produced within 24 hours, the drug is used again at the same doze. No drugs were applied to the animals in the control group.

When administering the drug no side effects and complications in the animals were observed.

The results are shown in Table 1.

### Example 3 (study of the drug contraceptive effect)

The contraceptive effect of the drug prepared according to the example 1 was studied in female rats, cats and dogs. The studies were performed on the basis of All-Russian Research Institute of Sheep and Goat Breeding, Stavropol, during the period from 2nd June to 30th June, 2014.

Female rats with the mass of 200-220 g were divided into 3 groups, 20 individuals in each. Apparently healthy animals previously kept in the 15-day-long quarantine were used for the study. In the first group the drug was applied to the skin on the rats' backs at the dose of 1 ml per animal once a day within two days. Male rats were placed next to the females after 5-7 days. Fertilized animals were determined by the presence of spermatozoa in the vaginal smears. The result is that 1 rat out of 20 animals was sired. Estrus retardation effect was 95.0%.

In the second group of rats with the signs of estrus the drug was once applied at the dose of 1 ml per rat. Male rats were placed next to them after 5-7 days. Pregnancy was determined after necropsy of the animals. The contraceptive activity of the drug was calculated by the number of nonpregnant rats out of the total number of rats copulated. The pregnancy was established in 1 rat out of 20 rats. Effectiveness of the contraceptive action was 95.0%.

**Table 1**

| The Drug Influence on Sexual Behavior of Animals as per Example 1 | | | | | |
|---|---|---|---|---|---|
| Test No. | Subject/quantity | Period | State when using the drug | Complete decrease of sexual arousal | Estrus recovery |
| 1. | **Cats/14** (Serpukhov Municipal Non-Governmental Charitable Foundation "Zoozashita Plus") | from February to May, 2014 | estrus | on 2-3 day | after 56-67 days |
| 2. | **Dogs/14** (Serpukhov Municipal Non-Governmental Charitable Foundation "Zoozashita Plus") | from February to May, 2014 | estrus | on 2-3 day | after 76 days estrus did not come |
| 3. | **Cats/12** (Balashikha Regional Station of Animals Disease Control) | from March to November, 2014 | estrus | on 2-3 day | after 2-4 months |
| 4. | **Dogs/8** (Balashikha Regional Station of Animals Disease Control) | from March to November, 2014 | estrus | on 2-3 day | after 3-6 months |
| 5. | **Cats/13** (Dzerzhinsky Station of Animals Disease Control) | from April to September, 2014 | estrus | on 1-3 day | after 2.5-3 months |
| 6. | **Dogs/11** (Dzerzhinsky Station of Animals Disease Control) | from April to September, 2014 | estrus | on 1-3 day | after 3-6 months |
| 7. | Cats/8 (State-Financed Institution of Pachelma Regional Station of Animals Disease Control, Penza) | from 25th February to 14th September, 2014 | estrus | on 1-2 day | after 2-3.5 months |
| 8. | **Dogs/6** (State-Financed Institution of Pachelma Regional Station of Animals Disease Control, Penza) | from 25th February to 14th September, 2014 | estrus | on 1-2 day | after 2-3.5 months |
| 9. | **Cats/8** (domestic outbred cats) | from May 2013 to January 2015 | estrus | on 1-2 day | after 4-5 months |
| 10. | **Dogs/4** (Ufa Municipal Veterinary Station) | from 1st October to 15th April, 2014 | 2 days of estrus 2 days of proestrus | on 3-6 day | after 4-6 months |
| 11. | **Cats/14** (Kaliningrad Veterinary Clinic No. 1) | from March to May, 2014 | estrus | on 2-3 day | after 52-60 days |
| 12. | **Dogs/14** (Kaliningrad Veterinary Clinic No. 1) | from March to May, 2014 | estrus | on 2-3 day | after 60 days estrus did not come |

In the third group of rats with the signs of estrus the drug was once applied at the dose of 1 ml per individual. Males were placed next to the females 2 days after application of the drug. Pregnancy was determined after necropsy of the animals. The contraceptive activity of the drug was calculated by the number of nonpregnant rats out of the total number of rats copulated. The pregnancy was established in one rat out of 20 rats. Effectiveness of the contraceptive action was 95.0%.

When studying the contraceptive action in dogs, the drug was once applied onto the back along the spinal cord between the scapulas of apparently healthy female dogs at the dose of 1 ml per 5 kg of body mass on the first/second day of the onset of the first signs of estrus. The use of the drug resulted in animals calming and estrus interruption in one dog on the 2nd day after application, in two dogs on the 3rd day, in three dogs on the 5th day.

When studying the contraceptive action in cats, the drug was once applied onto the back along the spinal cord between the scapulas of apparently healthy sexually mature female cats at the dose of 1 ml per 5 kg of body mass of an animal when the first signs of estrus appeared. The use of the drug resulted in animals calming and estrus interruption in one cat on the 3rd day after application, in two cats on the 4th day, and in three cats on the 5th day.

Conclusion. Study of the drug contraceptive action prepared in accordance with the example 1 in laboratory animals (rats), as well as cats and dogs, has shown the high efficiency of this agent application to interrupt estrus in females. Effectiveness of the contraceptive action of the drug was 95.0%. Contraceptive effect of the drug is long-term and constant enough (groups 2 and 3 of rats); with the purpose of contraception the drug may be used both before estrus onset (group 1 of rats) and during estrus development (groups 2 and 3 of rats).

### Example 4 (acute toxicity)

Parameters determination of acute toxicity of the drug prepared according to the example 1 was performed in accordance with the "Methodological Recommendations on Study of General Toxical Action of Pharmaceutical Substances", M.: "Minzdrav", 2005. 34 white rats with the mass of 180-200 g and 36 white mice with the mass of 21-23 g were taken for determination of the parameters of acute toxicity. Apparently healthy animals previously kept in the 15-day-long quarantine were taken for the experiment. Statistical groups consisted of 6 animals. Animals were injected intraperitoneally with the drug. Animals were monitored for 2 weeks after injection with noting the periods of animals death or recovery. Overall health of animals, preservation of movement functions, appetite, condition of hair coat, respiration, response to the exogenous irritants. The local action of the drug and its absorption through skin were also studied.

The following doses were applied to the mice during tests: 0.5; 1.0; 3.0; 5.0 and 6.0 g/kg. The obtained results are shown in Table 2.

**Table 2**

| Dose g/kg | 0.5 | 1.0 | 3.0 | 5.0 | 6.0 |
|---|---|---|---|---|---|
| Survived | 6 | 5 | 3 | 1 | 0 |
| Died | 0 | 1 | 3 | 5 | 6 |

LD₅₀ value was 3.0±0.54 g/kg. Using graphical method of "dose-effect" dependence analysis, the LD₁₆ value and LD₈₄ value were determined as 0.8 g/kg and 5.3 g/kg correspondingly. Thus, the drug relates to the mildly toxic compounds (class 3) in accordance with the classification of GOST 12.1.007-76.

The similar experiment was carried out on white outbred rats. The dose range of 3.0-14.0 g/kg of body mass was tested. The results are shown in Table 3.

**Table 3**

| Dose g/kg | 3.0 | 6.0 | 8.0 | 10.0 | 12.0 | 14.0 |
|---|---|---|---|---|---|---|
| Survived | 6 | 6 | 4 | 3 | 2 | 0 |
| Died | 0 | 0 | 2 | 3 | 4 | 6 |

LD₅₀ value was 9.8 g/kg. Using graphical method of "dose-effect" dependence analysis, the LD₁₆ value and LD₈₄ value were determined as 6.8 g/kg and 12.3 g/kg correspondingly. Thus, the drug may be designated as low-toxic compounds (class 4).

Obtained data allow calculating the species sensitivity coefficient in case of single intragastric administration. The coefficient for the studied drug is equal to 3.1 that demonstrates the moderate sensitivity of animals to the toxic action of the drug.

Clinical findings of intoxication do not depend on species of experimental animals and characterized by the apparent animal distress, respiration disturbance and dystaxia. Skin cover cyanosis, apastia during 1-2 days is observed in the animals. Death occurred in the setting of developed coma. Death of the animals was recorded during the first two days. Signs of toxicosis in the survived animals gradually faded away, but the hair was still untidy.

Absorption of the drug through skin was under study within the single and replicated (during 5 days) experiments on the white rats.

The purpose of the single experiment was determination of fatal doses and determination of cutaneous-oral coefficient correspondingly. For this purpose, the drug was 2 times applied to the clipped skin area on the rats' back at the volume of 1 ml (5 g/kg) within two hours. Total dose was 10 g/kg. The result was that the clinical findings of intoxication of animals received this dose had much in common with the control. The animals were active, lively and were well fed. Animal death was not observed. Similar data were obtained when applying the drug daily at the dose of 5.0 g/kg during five days.

Thus obtained data show that the toxic compounds of the drug scarcely absorbed through skin. Skin-irritating effect of the drug was not observed when the drug was applied once and repeatedly. No erythema of the skin, scratches, cutaneous dropsy or skin fold thickening were detected.

### Example 5 (drug tolerance, dogs)

The study was conducted on 9 female dogs of different breeds (outbred, Collie, Bull Terrier, Bulldog) aged from 1 to 3 years with the mass of 2.0-3.5 kg. Experimental animals were divided into three groups, three individuals in each: one control group and two experimental ones. The drug according to the example 1 was applied to the skin along the spinal cord of the animals of experimental groups at the dose of 1 dropper pipette (1 ml) per individual and 3 dropper pipettes (3 ml) per individual. No drugs were applied to the animals in the control group. All animals were kept under the same conditions throughout the period of the test. Clinical observations were made a day before application of the drug, and then 1, 2 and 5 days after its repeated application (within 10 days with breaks on the third and sixth days). Clinical examination of the animals included measurement of body temperature, counting of heart beats and respiratory movements, visual assessment of overall health and hematology values. The red blood count, white blood count, and level of hemoglobin were determined among hematology values. The blood for the studies was taken from the saphenous vein of the lower thigh. Clinical observations and blood sampling were conducted at the same time - in the morning before feeding the animals.

The results of study have shown that repeated application of the drug at the therapeutic dose as well as at the dose 3 times greater than the therapeutic dose did not cause any irregularities in terms of the physiological state of the animals. The temperature of the body ranged from 38.0 to 38.5°C with the normal range of 37.5-39.0°C. The pulse rate was within the range of 70-77 beats per minute (the normal range is 70-80 beats per minute). The quantity of respiratory movements ranged from 15 to 30 respiratory movements per minute (the normal range is 10-30). The overall health of the animals in the experimental groups was no different from that of the control individuals.

Tests have shown that animals tolerate adequately the drug according to the example 1 when the drug is applied repeatedly (within 10 days with breaks on the third and sixth days) to the female dogs of different breeds and age groups at the therapeutic dose as well as at the dose three times greater than the therapeutic dose, and the drug has no adverse effect on the organism's physiological state and hematology values (Table 4). Three-time dose of the applied drug causes animal distress and feed refusal in the last two days of the drug application.

**Table 4**

| Influence of the drug according to example 1 on morphological composition of blood in female dogs | | | |
|---|---|---|---|
| Group | Erythrocytes, 10¹² /l | Hemoglobin, g% | Leucocytes, 10⁹ /l |
| Control | 8.1±0.4 | 9.2±0.3 | 9.2±0.3 |
| Therapeutic dose | 8.5±0.4 | 9.8±0.1 | 9.8±0.3 |
| Three-time dose | 7.2±0.4 | 7.5±0.2 | 8.0±0.1 |

Conclusion: the drug according to the example 1 applied at the therapeutic dose as well as at the dose 3 times greater than the therapeutic dose has no adverse effect on the clinical state and hematology values of the female dogs.

### Example 6 (drug tolerance, cats)

For the purpose of studying the tolerance of the drug according to the example 1 tests have been conducted on 9 long-haired and short-haired cats with the mass 2.5-5.0 kg, aged from 15 months to 3.5 years. The cats were divided into three groups, three individuals in each: a control group and two experimental ones. In the first experimental group the drug was applied to the skin on the animals' back at the dose of 1 dropper pipette (1 ml) per individual daily during 8 days with breaks on the third and sixth days; in the second group the drug was applied at the dose of 3 dropper pipettes (3 ml) daily during 8 days with breaks on the third and sixth days. No drugs were applied to the cats in the control group. All animals were kept under the same conditions throughout the period of the test.

Clinical observations were made a day before application of the drug; 1, 6, 12 hours after and 1, 2, 5 days after completion of application of the drug. Assessment of the animals included clinical examination, measurement of body temperature, pulse and respiration rate, as well as hematology and blood chemistry values. The red blood count, white blood count, hemoglobin level and nuclear Arneth index were determined among hematology values. The blood for the studies was taken from the saphenous vein of the lower thigh. Clinical observations and blood sampling were conducted at the same time - in the morning before feeding the animals.

According to the results of the studies, cats tolerate adequately the drug when the drug is applied repeatedly at the therapeutic dose as well as at the dose 3 times greater than the therapeutic dose. No irregularities in terms of the physiological state of the animals have been detected, appetite preserved, the temperature of the body ranged from 38.5 to 39.0°C, the pulse rate was within the range 100-110 beats per minute (the normal range is 100-125), the respiration rate was 20-25 respiratory movements per minute (the normal range is 20-30). The overall health of the animals in the experimental groups was no different from that of the control ones.

The drug does not cause changes in the cats' hematology and blood chemistry values at the therapeutic dose. Exceeding of the therapeutic dose by three times leads to minor changes in the hematology and blood chemistry values (see Tables 5, 6).

**Table 5**

| Influence of the drug according to the example 1 on morphological composition of blood in cats. | | | |
|---|---|---|---|
| Group | Erythrocytes, 10¹²/l | Hemoglobin, g% | Leucocytes, 10⁹/l |
| Control | 7.8±0.3 | 9.4±0.1 | 10.0±0.2 |
| Therapeutic dose | 7.8±0.1 | 9.4±0.2 | 10.5±0.5 |
| 3 times greater than the therapeutic dose | 6.8±0.2 | 8.1±0.4 | 8.8±0.1 |

**Table 6**

| Blood chemistry values in cats | | | |
|---|---|---|---|
| Indicators | Control | Therapeutic dose | 3 times greater than the therapeutic dose |
| Protein, g/l | 55.0±0.4 | 52.0±0.7 | 49.0±0.2 |
| Albumin, g/l | 33.2±0.6 | 32.2±0.2 | 28.2±0.7 |
| Alkaline phosphatase, units/l | 62.8±2.2 | 69.7±1.8 | 65.4±2.8 |
| Amylase, units/l | 460.0±2.2 | 468.2±10.2 | 482.1±11.2 |
| AST, units/l | 28.8±0.4 | 28.6±0.4 | 26.4±0.7 |
| ALT, units/l | 26.8±0.4 | 26.2±0.6 | 24.0±0.2 |
| Glucose, mmol/l | 4.2±0.3 | 4.1±0.1 | 4.7±0.1 |

Conclusion: the drug according to the example 1 applied at the therapeutic dose as well as at the dose 3 times greater than the therapeutic dose has no adverse effect on the cats' clinical state, hematology and blood chemistry values.

### Example 7 (subchronic toxicity, rats)

The subchronic toxicity study of the drug according to the example 1 was conducted on 24 white outbred female rats. The animals were divided into three groups, 8 individuals in each group. The animals in the first experimental group was applied with the drug cutaneously to the back area during 45 days at the dose of 1/100 of the LD₅₀ of animal's body mass (0.09 g/kg of body mass); the animals in the second experimental group was applied with the drug at the dose of 1/1000 of the LD₅₀ of body mass of the animal (0.009 g/kg). No drugs were applied to the rats in the control group. All animals were kept under the same conditions throughout the period of the study.

Assessment of the animals included clinical examination, measurement of body mass, as well as hematology and blood chemistry values and the animals' urinalysis. The red blood count and white blood count, as well as the level of hemoglobin were determined among hematology studies. The blood for the studies was taken from the tail vein. Clinical observations and blood sampling were conducted at the same time - in the morning before feeding the animals.

The results of weighing of the animals are shown in Table 7.

**Table 7**

| Influence of the drug according to the example 1 on the mass gain of the rats on the 45^{th} day | | | |
|---|---|---|---|
| Weighing time | Body mass | | |
| | Control | 0.09 g/kg | 0.009 g/kg |
| 0 | 200.0±2.2 | 219.4±4.7 | 200.0±1.9 |
| Week 1 | 210.2±3.6 | 225.2±3.9 | 205.4±2.3 |
| Week 2 | 218.2±5.4 | 236.1±7.8 | 215.2±3.2 |

In the course of study of the hematology values (Table 8) it was found that the red blood count, white blood count, and hemoglobin level in the animals of both experimental groups were on the same level as in the animals of the control group. Blood sampling was conducted on the 45^{th} day of observations prior to terminal sacrifice.

**Table 8**

| Hematology values of the rats on the 45^{th} day from the date of start of application the drug according to the example 1 | | | | |
|---|---|---|---|---|
| Group | Hemoglobin, g/l | Erythrocytes, 10¹²/π | Leucocytes, 10⁹/l | ESR, mm/h |
| Control | 9.6±0.4 | 8.2±0.8 | 13.2±1.5 | 4.0 |
| 0.09 g/kg | 9.4±0.3 | 8.9±0.2 | 14.0±2.0 | 4.1 |
| 0.009 g/kg | 8.2±0.2 | 7.2±0.4 | 10.8±2.0 | 4.4 |

Table 8 contains the rats' blood chemistry values that characterize functional state of the liver.

Activity of alanine aminotransferase (ALT), aspartate aminotransferase (AST), total protein, uric acid and cholinesterase values in the animals in the experimental groups were not reliably different from those in the case of animals in the control group, however the blood chemistry values of the rats receiving the drug at the dose 0.009 g/kg were in the lower limit of the physiological range.

**Table 9**

| Blood chemistry values of rats on the 45^{th} day from the date of start of application the drug according to the example 1 | | | |
|---|---|---|---|
| Indicators | Control | 0.09 g/kg | 0.009 g/kg |
| ALT, units/l | 82.5±2.4 | 81.5±0.5 | 96.5±1.1 |
| AST, units/l | 95.8±4.5 | 98.2±3.3 | 112.8±7.2 |
| Total protein, g/l | 70.0±2.8 | 70.1±3.9 | 67.0±1.5 |
| Uric acid, mmol/l | 110.0±2.2 | 115.7±2.4 | 98.8±4.2 |
| Cholinesterase activity, mmol/l | 212.6±10.2 | 219.2±15.6 | 212.6±10.4 |

Thus, application of the drug at the doses 1/100 and 1/1000 of the LD₅₀ did not cause changes in the morphological composition and biochemistry values of the blood in animals used in the experiment compared to the control group. The drug did not affect blood coagulability or functional activity of the liver. The results of the urinalysis (daily diuresis was 4.6±0.1-5.2±0.4 ml/day) has shown that the average volume excreted by the experimental animals does not differ from that of the animals in the control group.

A conclusion can be made that the long-term drug application according to the example 1 at the doses of 1/100 and 1/1000 of LD₅₀ does not lead to intoxication of the laboratory animals and has no adverse effect on the physiological state and blood chemistry values.

### Example 8 (minimum quantity of megestrol)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is agitated when heated to 60°C till complete dissolution of the components. It is being cooled to the room temperature when agitated. pH value is adjusted to 4.29 by adding stearic acid with the control by an ion meter with input impedance of > 2 GOhm. The beaker is wrapped with nontransparent foil. 10 g of proroxan, 100 g of melatonin and 5.0 g of megestrol acetate are added to the obtained solution. The mass is being agitated when heated to 40°C till complete dissolution of the components. pH value is adjusted by adding stearic acid to the value of 4.32 with the control by an ion meter with input impedance of > 2 GOhm. The aggregate quantity of stearic acid used for pH level adjustment was 11 g. When cooled to the room temperature, the solution becomes cloudy. 991 g of transdermal composition with the following parameters are obtained:
1. Description - moving liquid cloudy yellow mass;
2. Density - 1.10 g/cm³;
3. pH 4.31;
4. Content of megestrol acetate is 5.42 mg/cm³;
5. Content of melatonin is 110.03 mg/cm³;
6. Content of proroxan is 11.07 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid mass, light-yellow solution;
2. Density - 1.11 g/cm³;
3. pH 4.29;
4. Content of megestrol acetate is 5.04 mg/cm³;
5. Content of melatonin is 110.89 mg/cm³;
6. Content of proroxan is 10.98 mg/cm³
7. Viscosity - 4.5 cPs.

Thus the drug is stable when stored under natural conditions in polymer pipettes for two years.

### Example 9 (maximum quantity of megestrol)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.32 by adding stearic acid with the control by an ion meter with input impedance of > 2 GOhm.

The beaker is wrapped with nontransparent foil. 10 g of proroxan, 100 g of melatonin and 18.5 g of megestrol acetate are added to the obtained solution. The mass is being agitated when heated to 40°C till complete dissolution of the components. pH value is adjusted by adding stearic acid to the value of 4.35 with the control by an ion meter with input impedance of > 2 GOhm. The aggregate quantity of stearic acid used for pH level adjustment was 11 g. When cooled to the room temperature, the solution becomes cloudy. 1004 g of transdermal composition with the following parameters are obtained.
1. Description - moving liquid cloudy yellow mass;
2. Density - 1.12 g/cm³;
3. pH 4.33;
4. Content of megestrol acetate is 20.01 mg/cm³;
5. Content of melatonin is 110.97 mg/cm³;
6. Content of proroxan is 11.02 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid mass, light-yellow solution;
2. Density - 1.09 g/cm³;
3. pH 4.30;
4. Content of megestrol acetate is 19.84 mg/cm³;
5. Content of melatonin is 109.21 mg/cm³;
6. Content of proroxan is 10.14 mg/cm³;
7. Viscosity - 4.5 cPs.

Thus, the drug is stable when stored under natural conditions in polymer pipettes for two years.

### Example 10 (average quantity of melatonin)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.29 by adding stearic acid with the control by an ion meter with input impedance of > 2 GOhm. The beaker is wrapped with nontransparent foil. 10 g of proroxan, 150 g of melatonin and 10.0 g of megestrol acetate are added to the obtained solution. The mass is being agitated when heated to 40°C till complete dissolution of the components. pH value is adjusted by adding stearic acid to the value of 4.30 with the control by an ion meter with input impedance of > 2 GOhm. The aggregate quantity of stearic acid used for pH level adjustment was 11 g. When cooled to the room temperature, the solution becomes cloudy. 1046 g of transdermal composition with the following parameters are obtained:
1. Description - moving liquid cloudy yellow mass;
2. Density - 1.14 g/cm³;
3. pH 4.29;
4. Content of megestrol acetate is 10.76 mg/cm³;
5. Content of melatonin is 163.24 mg/cm³;
6. Content of proroxan is 10.81 mg/cm³;
7. Viscosity - 4.5 cPs.

The drug is packed in polypropylene-polyvinyl chloride pipettes of 1 ml. After two years of storage the parameters of the drug are as follows:
1. Description - moving cloudy liquid light-yellow mass;
2. Density -1.14 g/cm³;
3. pH 4.30;
4. Content of megestrol acetate is 10.41 mg/cm³;
5. Content of melatonin is 162.74 mg/cm³;
6. Content of proroxan is 10.01 mg/cm³;
7. Viscosity - 4.5 cPs.

Thus the drug is stable when stored under natural conditions in polymer pipettes for two years.

### Example 11 (maximum quantity of melatonin)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.33 by adding stearic acid with the control by an ion meter with input impedance > 2 GOhm. The beaker is wrapped with nontransparent foil. 10 g of proroxan, 191 g of melatonin and 10.0 g of megestrol acetate are added to the obtained solution. The mass is being agitated when heated to 40°C till complete dissolution of the components. pH value is adjusted to 4.32 by adding stearic acid with the control by an ion meter with input impedance of > 2 GOhm. The aggregate quantity of stearic acid used for pH level adjustment was 11 g. When cooled to the room temperature, the solution becomes cloudy. 1087 g of transdermal composition with the following parameters are obtained:
1. Description - moving liquid cloudy yellow mass;
2. Density - 1.15 g/cm³;
3. pH 4.30;
4. Content of megestrol acetate is 10.51 mg/cm³;
5. Content of melatonin is 201.83 mg/cm³;
6. Content of proroxan is 10.71 mg/cm³;
7. Viscosity - 4.5 cPs.

The drug is packed in polypropylene-polyvinyl chloride pipettes of 1 ml. After two years of storage the parameters of the drug are as follows:
1. Description - moving cloudy liquid light-yellow mass;
2. Density -1.14 g/cm³;
3. pH 4.31;
4. Content of megestrol acetate is 10.11 mg/cm³;
5. Content of melatonin is 200.14 mg/cm³;
6. Content of proroxan is 10.21 mg/cm³;
7. Viscosity - 4.5 cPs.

Thus the drug is stable when stored under natural conditions in polymer pipettes for two years.

### Example 12 (average value of proroxan)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm. The beaker is wrapped with nontransparent foil. 15 g of proroxan, 100 g of melatonin and 10.0 g of megestrol acetate are added to the obtained solution. The mass is being agitated when heated to 40°C till complete dissolution of the components. pH value is adjusted by adding stearic acid to the value of 4.31 with the control by an ion meter with input impedance of > 2 GOhm. The aggregate quantity of stearic acid used for pH level adjustment was 11 g. When cooled to the room temperature, the solution becomes cloudy. 1001 g of transdermal composition with the following parameters are obtained:
1. Description - moving liquid cloudy yellow mass;
2. Density - 1.10 g/cm³;
3. pH 4.29;
4. Content of megestrol acetate is 10.64 mg/cm³;
5. Content of melatonin is 109.21 mg/cm³;
6. Content of proroxan is 16.21 mg/cm³
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass,
2. Density - 1.09 g/cm³;
3. pH 4.30;
4. Content of megestrol acetate is 10.03 mg/cm³;
5. Content of melatonin is 108.85 mg/cm³;
6. Content of proroxan is 15.94 mg/cm³;
7. Viscosity - 4.5 cPs.

Thus the drug is stable when stored under natural conditions in polymer pipettes for two years.

### Example 13 (maximum quantity of proroxan)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.29 by adding stearic acid with the control by an ion meter with input impedance of > 2 GOhm. The beaker is wrapped with nontransparent foil. 18 g of proroxan, 100 g of melatonin and 10.0 g of megestrol acetate are added to the obtained solution. The mass is being agitated when heated to 40°C till complete dissolution of the components. pH value is adjusted by adding stearic acid to the value of 4.30 with the control by an ion meter with input impedance of > 2 GOhm. The aggregate quantity of stearic acid used for pH level adjustment was 11 g. When cooled to the room temperature, the solution becomes cloudy. 1004 g of transdermal composition with the following parameters are obtained:
1. Description - moving liquid cloudy yellow mass;
2. Density - 1.11 g/cm³;
3. pH 4.30;
4. Content of megestrol acetate is 10.97 mg/cm³;
5. Content of melatonin is 110.21 mg/cm³;
6. Content of proroxan is 19.73 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid mass, light-yellow solution;
2. Density - 1.10 g/cm³;
3. pH 4.29;
4. Content of megestrol acetate is 10.41 mg/cm³;
5. Content of melatonin is 109.84 mg/cm³;
6. Content of proroxan is 19.14 mg/cm³;
7. Viscosity - 4.5 cPs.

Thus the drug is stable when stored under natural conditions in polymer pipettes for two years.

### Example 14 (progesterone)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.28 by adding stearic acid with the control by an ion meter with input impedance of > 2 GOhm. The beaker is wrapped with nontransparent foil. 10 g of proroxan, 100 g of melatonin and 10.0 g of progesterone are added to the obtained solution. The mass is being agitated when heated to 40°C till complete dissolution of the components. pH value is adjusted by adding stearic acid to the value of 4.29 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.12 g/cm³;
3. pH 4.30;
4. Content of progesterone is 11.12 mg/cm³;
5. Content of melatonin is 112.24 mg/cm³;
6. Content of proroxan is 11.17 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.11 g/cm³;
3. pH 4.28;
4. Content of progesterone is 10.89 mg/cm³;
5. Content of melatonin is 111.74 mg/cm³;
6. Content of proroxan is 11.01 mg/cm³;
7. Viscosity - 4.5 cPs.

Thus the drug is stable when stored under natural conditions in polymer pipettes for two years.

### Example 15 (oxyprogesterone capronate)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm. The beaker is wrapped with nontransparent foil. 10 g of proroxan, 100 g of melatonin and 10.0 g of oxyprogesterone capronate are added to the obtained solution. The mass is being agitated when heated to 40°C till complete dissolution of the components. pH value is adjusted by adding stearic acid to the value of 4.33 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.16 g/cm³;
3. pH 4.30;
4. Content of oxyprogesterone capronate is 11.54 mg/cm³;
5. Content of melatonin is 115.02 mg/cm³;
6. Content of proroxan is 11.35 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.13 g/cm³;
3. pH 4.26;
4. Content of oxyprogesterone capronate is 11.13 mg/cm³;
5. Content of melatonin is 114.43 mg/cm³;
6. Content of proroxan is 10.97 mg/cm³;
7. Viscosity - 4.5 cPs.

Thus the drug is stable when stored under natural conditions in polymer pipettes for 2 years.

### Example 16 (dydrogesterone)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.31 by adding stearic acid with the control by an ion meter with input impedance of > 2 GOhm. The beaker is wrapped with nontransparent foil. 10 g of proroxan, 100 g of melatonin and 10 g of dydrogesterone are added to the obtained solution. The mass is being agitated when heated to 40°C till complete dissolution of the components. pH value is adjusted by adding stearic acid to the value of 4.31 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.12 g/cm³;
3. pH 4.30;
4. Content of dydrogesterone is 11.07 mg/cm³;
5. Content of melatonin is 112.21 mg/cm³;
6. Content of proroxan is 11.14 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid mass, light-yellow solution;
2. Density - 1.11 g/cm³;
3. pH 4.27;
4. Content of dydrogesterone is 10.87 mg/cm³;
5. Content of melatonin is 111.97 mg/cm³;
6. Content of proroxan is 10.91 mg/cm³;
7. Viscosity - 4.5 cPs.

Thus the drug is stable when stored under natural conditions in polymer pipettes for 2 years.

### Example 17 (pregninum)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.29 by adding stearic acid with the control by an ion meter with input impedance of > 2 GOhm. The beaker is wrapped with nontransparent foil. 10 g of proroxan, 100 g of melatonin and 10 g of pregninum are added to the obtained solution. The mass is being agitated when heated to 40°C till complete dissolution of the components. pH value is adjusted by adding stearic acid to the value of 4.31 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.10 g/cm³;
3. pH 4.31;
4. Content of pregninum is 10.93 mg/cm³;
5. Content of melatonin is 111.01 mg/cm³;
6. Content of proroxan is 10.87 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.12 g/cm³;
3. pH 4.29;
4. Content of pregninum is 10.42 mg/cm³;
5. Content of melatonin is 110.05 mg/cm³;
6. Content of proroxan is 10.37 mg/cm³;
7. Viscosity - 4.5 cPs.

Thus the drug is stable when stored under natural conditions in polymer pipettes for 2 years.

### Example 18 (norethisterone)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.27 by adding stearic acid with the control by an ion meter with input impedance of > 2 GOhm. The beaker is wrapped with nontransparent foil. 10 g of proroxan, 100 g of melatonin and 10 g of norethisterone are added to the obtained solution. The mass is being agitated when heated to 40°C till complete dissolution of the components. pH value is adjusted by adding stearic acid to the value of 4.29 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.11 g/cm³;
3. pH 4.31;
4. Content of norethisterone is 11.03 mg/cm³;
5. Content of melatonin is 110.57 mg/cm³;
6. Content of proroxan is 10.98 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.11 g/cm³;
3. pH 4.33;
4. Content of norethisterone is 10.73 mg/cm³;
5. Content of melatonin is 110.02 mg/cm³;
6. Content of proroxan is 10.46 mg/cm³;
7. Viscosity - 4.5 cPs.

Thus the drug is stable when stored under natural conditions in polymer pipettes for 2 years.

### Example 19 (levonorgestrel)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm. The beaker is wrapped with nontransparent foil. 10 g of proroxan, 100 g of melatonin and 10 g of levonorgestrel are added to the obtained solution. The mass is being agitated when heated to 40°C till complete dissolution of the components. pH value is adjusted by adding stearic acid to the value of 4.28 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.10 g/cm³;
3. pH 4.30;
4. Content of levonorgestrel is 10.86 mg/cm³;
5. Content of melatonin is 110.99 mg/cm³;
6. Content of proroxan is 11.02 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.11 g/cm³;
3. pH 4.29;
4. Content of levonorgestrel is 10.42 mg/cm³;
5. Content of melatonin is 110.51 mg/cm³;
6. Content of proroxan is 10.83 mg/cm³;
7. Viscosity - 4.5 cPs.

Thus the drug is stable when stored under natural conditions in polymer pipettes for 2 years.

### Example 20 (allylestrenol)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.29 by adding stearic acid with the control by an ion meter with input impedance of > 2 GOhm. The beaker is wrapped with nontransparent foil. 10 g of proroxan, 100 g of melatonin and 10 g of allylestrenol are added to the obtained solution. The mass is being agitated when heated to 40°C till complete dissolution of the components. pH value is adjusted by adding stearic acid to the value of 4.32 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.12 g/cm³;
3. pH 4.31;
4. Content of allylestrenol is 11.12 mg/cm³;
5. Content of melatonin is 112.31 mg/cm³;
6. Content of proroxan is 11.19 mg/cm³;
7. Viscosity - 4.5 cPs.

The drug is packed in polypropylene-polyvinyl chloride pipettes of 1 ml. After 2 years of storage the parameters of the drug are as follows.
1. Description - moving cloudy liquid light-yellow mass;
2. Density - 1.10 g/cm³;
3. pH 4.30;
4. Content of allylestrenol is 10.80 mg/cm³;
5. Content of melatonin is 112.07 mg/cm³;
6. Content of proroxan is 10.84 mg/cm³;
7. Viscosity - 4.5 cPs.

Thus the drug is stable when stored under natural conditions in polymer pipettes for 2 years.

### Example 21 (lynestrenol)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.29 by adding stearic acid with the control by an ion meter with input impedance of > 2 GOhm. The beaker is wrapped with nontransparent foil. 10 g of proroxan, 100 g of melatonin and 10 g of lynestrenol are added to the obtained solution. The mass is being agitated when heated to 40°C till complete dissolution of the components. pH value is adjusted by adding stearic acid to the value of 4.31 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.10 g/cm³;
3. pH 4.33;
4. Content of lynestrenol is 10.87 mg/cm³;
5. Content of melatonin is 110.04 mg/cm³;
6. Content of proroxan is 10.91 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.11 g/cm³;
3. pH 4.31;
4. Content of lynestrenol is 10.47 mg/cm³;
5. Content of melatonin is 109.78 mg/cm³;
6. Content of proroxan is 10.75 mg/cm³;
7. Viscosity - 4.5 cPs.

Thus the drug is stable when stored under natural conditions in polymer pipettes for 2 years.

### Example 22 (acetomepregenol)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components.

It is being cooled to the room temperature when agitated. pH value is adjusted to 4.28 by adding stearic acid with the control by an ion meter with input impedance of > 2 GOhm. The beaker is wrapped with nontransparent foil. 10 g of proroxan, 100 g of melatonin and 10 g of acetomepregenol are added to the obtained solution. The mass is being agitated when heated to 40°C till complete dissolution of the components. pH value is adjusted by adding stearic acid to the value of 4.32 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.09 g/cm³;
3. pH 4.30;
4. Content of acetomepregenol is 10.82 mg/cm³;
5. Content of melatonin is 109.31 mg/cm³;
6. Content of proroxan is 10.80 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.11 g/cm³;
3. pH 4.29;
4. Content of acetomepregenol is 10.12 mg/cm³;
5. Content of melatonin is 109.04 mg/cm³;
6. Content of proroxan is 10.54 mg/cm³;
7. Viscosity - 4.5 cPs.

Thus the drug is stable when stored under natural conditions in polymer pipettes for 2 years.

### Example 23 (gestodene, pyruvic acid)

404 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.28 by adding pyruvic acid with the control by an ion meter with input impedance of > 2 GOhm. The beaker is wrapped with nontransparent foil. 10 g of proroxan, 100 g of melatonin and 10 g of gestodene are added to the obtained solution. The mass is being agitated when heated to 40°C till complete dissolution of the components. pH value is adjusted by adding pyruvic acid to the value of 4.33 with the control by an ion meter with input impedance of > 2 GOhm. The aggregate quantity of pyruvic acid used for pH level adjustment was 1.1 g. When cooled to the room temperature, the solution becomes a little cloudy. 996 g of transdermal composition with the following parameters are obtained.
1. Description - moving liquid cloudy yellow mass;
2. Density - 1.11 g/cm³;
3. pH 4.31;
4. Content of gestodene is 11.02 mg/cm³;
5. Content of melatonin is 110.91 mg/cm³;
6. Content of proroxan is 10.98 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.10 g/cm³;
3. pH 4.28;
4. Content of gestodene is 10.72 mg/cm³;
5. Content of melatonin is 109.74 mg/cm³;
6. Content of proroxan is 10.21 mg/cm³;
7. Viscosity - 4.5 cPs.

Thus the drug is stable when stored under natural conditions in polymer pipettes for 2 years.

### Example 24 (dienogest)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm. The beaker is wrapped with nontransparent foil. 10 g of proroxan, 100 g of melatonin and 10 g of dienogest are added to the obtained solution. The mass is being agitated when heated to 40°C till complete dissolution of the components. pH value is adjusted by adding stearic acid to the value of 4.28 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.09 g/cm³;
3. pH 4.29;
4. Content of dienogest is 10.87 mg/cm³;
5. Content of melatonin is 109.13 mg/cm³;
6. Content of proroxan is 10.61 mg/cm³;
7. Viscosity - 4.5 cPs.

The drug is packed in polypropylene-polyvinyl chloride pipettes of 1 ml. After 2 years of storage the parameters of the drug are as follows.
1. Description - moving cloudy liquid light-yellow mass;
2. Density - 1.08 g/cm³;
3. pH 4.31;
4. Content of dienogest is 10.42 mg/cm³;
5. Content of melatonin is 108.86 mg/cm³;
6. Content of proroxan is 10.11 mg/cm³;
7. Viscosity - 4.5 cPs.

Thus the drug is stable when stored under natural conditions in polymer pipettes for 2 years.

### Example 25 (drospirenone, stearic acid + succinic acid)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.25 by adding stearic acid with the control by an ion meter with input impedance of > 2 GOhm. The beaker is wrapped with nontransparent foil. 10 g of proroxan, 100 g of melatonin and 10 g of drospirenone are added to the obtained solution. The mass is being agitated when heated to 40°C till complete dissolution of the components. pH value is adjusted by adding succinic acid to the value of 4.26 with the control by an ion meter with input impedance of > 2 GOhm. The aggregate quantity of stearic acid used for pH level adjustment was 10.0 g, succinic acid - 160 mg. When cooled to the room temperature, the solution becomes cloudy. 996 g of transdermal composition with the following parameters are obtained:
1. Description - moving liquid cloudy yellow mass;
2. Density - 1.10 g/cm³;
3. pH 4.28;
4. Content of drospirenone is 10.92 mg/cm³;
5. Content of melatonin is 110.14 mg/cm³;
6. Content of proroxan is 10.85 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.09 g/cm³;
3. pH 4.26;
4. Content of drospirenone is 10.12 mg/cm³;
5. Content of melatonin is 109.94 mg/cm³;
6. Content of proroxan is 10.31 mg/cm³;
7. Viscosity - 4.5 cPs.

Thus the drug is stable when stored under natural conditions in polymer pipettes for 2 years.

### Example 26 (gestonorone capronate)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.31 by adding stearic acid with the control by an ion meter with input impedance of > 2 GOhm. The beaker is wrapped with nontransparent foil. 10 g of proroxan, 100 g of melatonin and 10 g of gestonorone capronate are added to the obtained solution. The mass is being agitated when heated to 40°C till complete dissolution of the components. pH value is adjusted by adding stearic acid to the value of 4.32 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.12 g/cm³;
3. pH 4.29;
4. Content of gestonorone capronate is 11.03 mg/cm³;
5. Content of melatonin is 112.37 mg/cm³;
6. Content of proroxan is 11.16 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.10 g/cm³;
3. pH 4.32;
4. Content of gestonorone capronate is 10.12 mg/cm³;
5. Content of melatonin is 109.94 mg/cm³;
6. Content of proroxan is 10.31 mg/cm³;
7. Viscosity - 4.5 cPs.

Thus the drug is stable when stored under natural conditions in polymer pipettes for 2 years.

### Example 27 (medroxyprogesterone)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components.

It is being cooled to the room temperature when agitated. pH value is adjusted to 4.33 by adding stearic acid with the control by an ion meter with input impedance of > 2 GOhm. The beaker is wrapped with nontransparent foil. 10 g of proroxan, 100 g of melatonin and 10 g of medroxyprogesterone are added to the obtained solution. The mass is being agitated when heated to 40°C till complete dissolution of the components. pH value is adjusted by adding stearic acid to the value of 4.35 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.11 g/cm³;
3. pH 4.34;
4. Content of medroxyprogesterone 11.01 mg/cm³;
5. Content of melatonin is 111.24 mg/cm³;
6. Content of proroxan is 11.10 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.09 g/cm³;
3. pH 4.32;
4. Content of medroxyprogesterone is 10.54 mg/cm³;
5. Content of melatonin is 110.83 mg/cm³;
6. Content of proroxan is 10.77 mg/cm³;
7. Viscosity - 4.5 cPs.

Thus the drug is stable when stored under natural conditions in polymer pipettes for 2 years.

### Example 28 (mepregenol acetate)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm. The beaker is wrapped with nontransparent foil. 10 g of proroxan, 100 g of melatonin and 10.0 g of mepregenol acetate are added to the obtained solution. The mass is being agitated when heated to 40°C till complete dissolution of the components. pH value is adjusted by adding stearic acid to the value of 4.28 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.08 g/cm³;
3. pH 4.32;
4. Content of mepregenol acetate is 10.61 mg/cm³;
5. Content of melatonin is 108.19 mg/cm³;
6. Content of proroxan is 10.73 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.10 g/cm³;
3. pH 4.29;
4. Content of mepregenol acetate is 10.45 mg/cm³;
5. Content of melatonin is 108.01 mg/cm³;
6. Content of proroxan is 10.57 mg/cm³;
7. Viscosity - 4.5 cPs.

Thus the drug is stable when stored under natural conditions in polymer pipettes for 2 years.

### Example 29 (mepregenol acetate butyrate)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.33 by adding stearic acid with the control by an ion meter with input impedance of > 2 GOhm. The beaker is wrapped with nontransparent foil. 10 g of proroxan, 100 g of melatonin and 10 g of mepregenol acetate butyrate are added to the obtained solution. The mass is being agitated when heated to 40°C till complete dissolution of the components. pH value is adjusted by adding stearic acid to the value of 4.30 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.10 g/cm³;
3. pH 4.31;
4. Content of mepregenol acetate butyrate is 10.71 mg/cm³;
5. Content of melatonin is 110.21 mg/cm³;
6. Content of proroxan is 10.97 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.09 g/cm³;
3. pH 4.28;
4. Content of mepregenol acetate butyrate is 10.14 mg/cm³;
5. Content of melatonin is 109.97 mg/cm³;
6. Content of proroxan is 10.48 mg/cm³;
7. Viscosity - 4.5 cPs.

Thus the drug is stable when stored under natural conditions in polymer pipettes for 2 years.

### Example 30 (mepregenol acetate hemisuccinate)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 6.0 by adding stearic acid with the control by an ion meter with input impedance of > 2 GOhm. The beaker is wrapped with nontransparent foil. 10 g of proroxan, 100 g of melatonin and 10 g of mepregenol acetate hemisuccinate are added to the obtained solution. The mass is being agitated when heated to 40°C till complete dissolution of the components. pH value is adjusted by adding stearic acid to the value of 6.0 with the control by an ion meter with input impedance of > 2 GOhm. The aggregate quantity of stearic acid used for pH level adjustment was 8.8 g. When cooled to the room temperature, the solution becomes cloudy. 996 g of transdermal composition with the following parameters are obtained:
1. Description - moving liquid cloudy yellow mass;
2. Density - 1.09 g/cm³;
3. pH 6.0;
4. Content of mepregenol acetate hemisuccinate is 10.64 mg/cm³;
5. Content of melatonin is 109.12 mg/cm³;
6. Content of proroxan is 10.77 mg/cm³;
7. Viscosity - 4.5 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.10 g/cm³;
3. pH 6.02;
4. Content of mepregenol acetate hemisuccinate is 10.34 mg/cm³;
5. Content of melatonin is 108.92 mg/cm³;
6. Content of proroxan is 10.36 mg/cm³;
7. Viscosity - 4.5 cPs.

Thus the drug is stable when stored under natural conditions in polymer pipettes for 2 years.

### Example 31 (medical spray)

266 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl) octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.31 by adding myristic acid with the control by an ion meter with input impedance of > 2 GOhm. The beaker is wrapped with nontransparent foil. 20 g of proroxan, 200 g of melatonin and 20 g of megestrol acetate are added to the obtained solution. The mass is being agitated when heated to 40°C till complete dissolution of the components. "Lemon Bouquet" essence 0.2 g is added. pH value is adjusted by adding myristic acid to the value of 4.34 with the control by an ion meter with input impedance of > 2 GOhm. The aggregate quantity of myristic acid used for pH level adjustment was 22 g. When cooled to the room temperature, the solution becomes cloudy. 997 g of transdermal composition with the following parameters are obtained:
1. Description - moving liquid cloudy yellow mass with pleasant scent;
2. Density - 1.15 g/cm³;
3. pH 4.40;
4. Content of megestrol acetate is 22.42 mg/cm³;
5. Content of melatonin is 229.91 mg/cm³;
6. Content of proroxan is 22.84 mg/cm³;
7. Viscosity - 4.6 cPs.

The drug is packed in 10 ml bottles fitted with a mechanical sprayer. After 2 years of storage the parameters of the drug are as follows:
1. Description - moving cloudy liquid light-yellow mass with pleasant scent;
2. Density - 1.14 g/cm³;
3. pH 4.33;
4. Content of megestrol acetate is 21.87 mg/cm³;
5. Content of melatonin is 229.11 mg/cm³;
6. Content of proroxan is 22.43 mg/cm³;
7. Viscosity - 4.6 cPs.

Thus the drug is stable when stored under natural conditions in polymer bottles for 2 years.

### Example 32 (without proroxan)

411 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm. The beaker is wrapped with nontransparent foil. 100 g of melatonin and 10 g of megestrol acetate are added to the obtained solution. The mass is being agitated when heated to 40°C till complete dissolution of the components. pH value is adjusted by adding stearic acid to the value of 4.25 with the control by an ion meter with input impedance of > 2 GOhm. The aggregate quantity of stearic acid used for pH level adjustment was 4 g. When cooled to the room temperature, the solution becomes cloudy. 995 g of transdermal composition with the following parameters are obtained:
1. Description - moving liquid cloudy yellow mass;
2. Density - 1.13 g/cm³;
3. pH 4.33;
4. Content of megestrol acetate is 11.12 mg/cm³;
5. Content of melatonin is 112.78 mg/cm³;
6. Viscosity - 4.6 cPs.

The drug is packed in polypropylene-polyvinyl chloride pipettes of 1 ml.

### Example 33 (non-selective α-adrenergic blocking agent - tropaphenum)

389 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.28 by adding stearic acid with the control by an ion meter with input impedance of > 2 GOhm. The beaker is wrapped with nontransparent foil. 15 g of tropaphenum, 100 g of melatonin and 10 g of megestrol acetate are added to the obtained solution. The mass is being agitated when heated to 40°C till complete dissolution of the components. pH value is adjusted by adding stearic acid to the value of 4.31 with the control by an ion meter with input impedance of > 2 GOhm. The aggregate quantity of stearic acid used for pH level adjustment was 9 g. When cooled to the room temperature, the solution becomes cloudy. 994 g of transdermal composition with the following parameters are obtained:
1. Description - moving liquid cloudy yellow mass;
2. Density - 1.12 g/cm³;
3. pH 4.33;
4. Content of megestrol acetate is 11.08 mg/cm³;
5. Content of melatonin is 112.58 mg/cm³;
6. Content of tropaphenum is 16.18 mg/cm³;
7. Viscosity - 4.6 cPs.

The drug is packed in polypropylene-polyvinyl chloride pipettes of 1 ml.

### Example 34 (selective α-adrenergic blocking agent - prazosin)

401 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.30 by adding stearic acid with the control by an ion meter with input impedance of > 2 GOhm.

The beaker is wrapped with nontransparent foil. 2.5 g of prazosin, 100 g of melatonin and 10 g of megestrol acetate are added to the obtained solution. The mass is being agitated when heated to 40°C till complete dissolution of the components. pH value is adjusted by adding stearic acid to the value of 4.28 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.10 g/cm³;
3. pH 4.31;
4. Content of megestrol acetate is 11.03 mg/cm³;
5. Content of melatonin is 112.41 mg/cm³;
6. Content of prazosin is 2.41 mg/cm³;
7. Viscosity - 4.6 cPs.

The drug is packed in polypropylene-polyvinyl chloride pipettes of 1 ml.

### Example 35 (study of the drugs' effect: with proroxan, without proroxan, with tropaphenum, with prazosin)

The effects of the drugs prepared according to examples 1, 32, 33 and 34 were studied on 25 sexually mature apparently healthy female cats at the age of 2-5 years divided into 5 groups, 5 individuals in each group. The cats were fed according to a standard food ration using dry food, canned goods and sufficient amount of drinking water. The drugs were used once at the dosage of 1 ml per individual by application on intact skin along the spinal cord between the scapulas.
Group I was administered the drug according to the example 1,
Group II - in accordance with example 32,
Group III - in accordance with example 33,
Group IV - in accordance with example 34.

No drugs were applied to the control group.

The drug was used on animals at their first days of estrus which was evidenced by estradiol and progesterone values in the blood of the animals.

In the course of the experiment the content of estradiol and progesterone in the blood of the experimental animals was studied.

The content of estradiol in the blood of animals was defined using radioimmune chemiluminescent method. Venous blood sampling was performed prior to feeding in the mornings in the amount of 1-2 ml every 0, 60 and 90 minutes upon intravenous administration of gonadotropin-releasing hormone at a dose of 0.02-0.03 µg/kg. Blood samples were stored in the refrigerator. Whole blood samples were used for serum preparation.

The content of progesterone in the blood of animals was defined using radioimmune method. Venous blood sampling was performed prior to feeding in the mornings in the amount of 1-3 ml. Blood samples were stored in the refrigerator. Whole blood samples were used for serum preparation.

### Study results

Study results are shown in tables 10 and 11.

**Table 10**

| Content of estradiol in the blood, pg/ml | | | | | | | | |
|---|---|---|---|---|---|---|---|---|
| Group | No. | Up to | After 1 month | After 2 months | After 3 months | After 4 months | After 5 months | After 6 months |
| Control | 1 | 87.7 | 78.1 | 84.6 | 86.7 | 97.5 | 92.0 | 92.5 |
| | 2 | 92.2 | 85.1 | 86.5 | 82.7 | 98.6 | 87.3 | 89.9 |
| | 3 | 81.3 | 92.9 | 87.5 | 84.1 | 69.8 | 75.0 | 66.9 |
| | 4 | 78.6 | 81.9 | 87.6 | 87.1 | 68.3 | 67.3 | 86.6 |
| | 5 | 85.0 | 89.7 | 91.2 | 87.6 | 78.3 | 84.0 | 97.5 |
| Group I | 1 | 73.6 | 12.4 | 10.7 | 11.6 | 12.7 | 10.1 | 13.2 |
| | 2 | 91.6 | 10.1 | 12.4 | 11.3 | 11.9 | 10.8 | 12.9 |
| | 3 | 94.7 | 13.2 | 12.8 | 13.4 | 13.8 | 13.5 | 14.8 |
| | 4 | 81.7 | 14.1 | 13.7 | 12.6 | 13.1 | 13.4 | 13.6 |
| | 5 | 82.9 | 11.3 | 12.4 | 12.3 | 11.2 | 12.8 | 13.1 |
| Group II | 1 | 87.5 | 28.4 | 78.8 | 69.9 | 76.6 | 77.6 | 89.0 |
| | 2 | 68.3 | 32.3 | 93.1 | 68.3 | 82.9 | 79.5 | 85.0 |
| | 3 | 92.0 | 26.1 | 71.7 | 82.5 | 98.1 | 73.0 | 92.4 |
| | 4 | 97.5 | 26.6 | 85.5 | 86.5 | 84.6 | 71.6 | 96.5 |
| | 5 | 78.3 | 28.8 | 72.4 | 88.7 | 89.0 | 69.7 | 72.0 |
| Group III | 1 | 91.2 | 21.2 | 76.4 | 79.4 | 96.7 | 78.5 | 74.3 |
| | 2 | 68.3 | 27.6 | 67.4 | 74.9 | 85.4 | 87.3 | 81.7 |
| | 3 | 86.5 | 25.6 | 72.0 | 88.0 | 83.4 | 86.4 | 78.6 |
| | 4 | 87.7 | 30.0 | 67.0 | 71.7 | 80.3 | 81.0 | 95.0 |
| | 5 | 86.6 | 21.7 | 72.2 | 72.5 | 92.8 | 73.5 | 84.6 |
| Group IV | 1 | 90.7 | 24.1 | 82.5 | 96.1 | 87.8 | 77.8 | 88.1 |
| | 2 | 87.8 | 21.3 | 71.9 | 72.1 | 70.0 | 86.2 | 80.3 |
| | 3 | 89.8 | 20.8 | 89.1 | 83.9 | 80.0 | 81.1 | 79.8 |
| | 4 | 90.5 | 33.6 | 74.0 | 78.7 | 91.1 | 96.4 | 72.6 |
| | 5 | 88.2 | 21.2 | 83.4 | 95.3 | 98.0 | 75.2 | 93.0 |

**Table 11**

| Content of progesterone in the blood, nmole/ml | | | | | | | | |
|---|---|---|---|---|---|---|---|---|
| Group | No. | Up to | After 1 month | After 2 months | After 3 months | After 4 months | After 5 months | After 6 months |
| Control | 1 | 6.78 | 8.51 | 14.61 | 8.97 | 7.58 | 5.81 | 6.47 |
| | 2 | 10.17 | 8.46 | 8.39 | 11.73 | 9.87 | 8.48 | 6.34 |
| | 3 | 11.89 | 8.79 | 7.05 | 14.08 | 6.22 | 10.39 | 6.06 |
| | 4 | 7.44 | 10.52 | 7.91 | 18.48 | 6.41 | 19.03 | 8.23 |
| | 5 | 7.86 | 14.20 | 7.26 | 15.26 | 7.22 | 14.98 | 7.06 |
| Group I | 1 | 12.68 | 1.27 | 1.30 | 1.05 | 1.18 | 1.24 | 1.01 |
| | 2 | 6.85 | 1.31 | 1.34 | 0.98 | 1.11 | 1.02 | 1.36 |
| | 3 | 9.82 | 0.75 | 0.92 | 1.07 | 1.34 | 1.17 | 1.09 |
| | 4 | 8.67 | 1.21 | 1.34 | 0.97 | 0.78 | 1.02 | 1.28 |
| | 5 | 14.88 | 1.07 | 0.88 | 0.85 | 0.91 | 1.15 | 1.06 |
| Group II | 1 | 7.48 | 1.47 | 7.21 | 8.18 | 7.39 | 6.48 | 11.61 |
| | 2 | 8.95 | 1.08 | 9.46 | 11.03 | 11.74 | 11.17 | 10.14 |
| | 3 | 8.15 | 1.35 | 9.39 | 6.11 | 8.45 | 8 | 6.97 |
| | 4 | 10.18 | 0.87 | 8.82 | 10.47 | 9.02 | 10.53 | 11.15 |
| | 5 | 9.54 | 1.31 | 6.15 | 11.25 | 11.2 | 6.66 | 9.23 |
| Group III | 1 | 10.08 | 0.99 | 11.92 | 9.74 | 9.96 | 6.43 | 9.81 |
| | 2 | 10.31 | 1.02 | 10.39 | 6.15 | 6.45 | 7.22 | 10.59 |
| | 3 | 9.87 | 0.93 | 10.48 | 6.54 | 7.5 | 9.24 | 9.34 |
| | 4 | 11.99 | 0.88 | 6.03 | 6.92 | 7.36 | 8.12 | 6.44 |
| | 5 | 10.20 | 0.98 | 11.47 | 8.47 | 10.11 | 7.41 | 11.66 |
| Group IV | 1 | 8.81 | 0.83 | 10.3 | 7.14 | 8.07 | 9.3 | 11.7 |
| | 2 | 7.77 | 1.03 | 10.18 | 8.29 | 9.64 | 6.38 | 7.2 |
| | 3 | 9.31 | 1.34 | 10.26 | 10.81 | 11 | 12 | 9.71 |
| | 4 | 9.35 | 1.12 | 10.17 | 8.39 | 9.49 | 8.53 | 8.88 |
| | 5 | 10.37 | 1.22 | 10.05 | 6.31 | 6.53 | 8.24 | 7.31 |

Conclusion: the provided results show that application of the drug according to the example 1 has a regulatory impact on sexual behaviour, reducing the level of estradiol and progesterone to the basal level, corresponding to the level of anestrus for a period of up to 6 months. Application of the drugs according to examples 32, 33 and 34 also reduces levels of the studied hormones to the basal level; this effect, however, lasts for 1 month only, upon expiry of which their concentrations increase up to the level corresponding to proestrus phase. Application of the drugs according to examples 32, 33 and 34 is followed by animal distress and hypotonia.

### Example 36 (butyroxan)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.00 by adding stearic acid with the control by an ion meter with input impedance of > 2 GOhm. The beaker is wrapped with nontransparent foil. 10 g of butyroxan, 100 g of melatonin and 10 g of megestrol acetate are added to the obtained solution. The mass is being agitated when heated to 40°C till complete dissolution of the components. pH value is adjusted by adding stearic acid to the value of 4.00 with the control by an ion meter with input impedance of > 2 GOhm. The aggregate quantity of stearic acid used for pH level adjustment was 11 g. When cooled to the room temperature, the solution becomes cloudy. 996 g of transdermal composition with the following parameters are obtained:
1. Description - moving liquid cloudy yellow mass;
2. Density - 1.12 g/cm³;
3. pH 4.01;
4. Content of megestrol acetate is 11.06 mg/cm³;
5. Content of melatonin is 111.24 mg/cm³;
6. Content of proroxan is 11.01 mg/cm³;
7. Viscosity - 4.6 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.10 g/cm³;
3. pH 4.05;
4. Content of megestrol acetate is 10.21 mg/cm³;
5. Content of melatonin is 110.48 mg/cm³;
6. Content of proroxan is 10.82 mg/cm³
7. Viscosity - 4.6 cPs.

Thus the drug is stable when stored under natural conditions in polymer pipettes for 2 years.

### Example 37 (proroxan+butyroxan)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 4.40 by adding stearic acid with the control by an ion meter with input impedance of > 2 GOhm. The beaker is wrapped with nontransparent foil. 5 g of butyroxan, 5 g of proroxan, 100 g of melatonin and 10 g of megestrol acetate are added to the obtained solution. The mass is being agitated when heated to 40°C till complete dissolution of the components. pH value is adjusted by adding stearic acid to the value of 4.33 with the control by an ion meter with input impedance of > 2 GOhm. The aggregate quantity of stearic acid used for pH level adjustment was 11 g. When cooled to the room temperature, the solution becomes cloudy. 995 g of transdermal composition with the following parameters are obtained:
1. Description - moving liquid cloudy yellow mass;
2. Density - 1.10 g/cm³;
3. pH 4.31;
4. Content of megestrol acetate is 11.07 mg/cm³;
5. Content of melatonin is 111.05 mg/cm³;
6. Content of proroxan is 5.40 mg/cm³;
7. Content of butyroxan is 5.31 mg/cm³;
8. Viscosity - 4.6 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.10 g/cm³;
3. pH 4.38;
4. Content of megestrol acetate is 10.41 mg/cm³;
5. Content of melatonin is 110.74 mg/cm³;
6. Content of proroxan is 5.21 mg/cm³;
7. Content of butyroxan is 5.18 mg/cm³;
8. Viscosity - 4.6 cPs.

Thus the drug is stable when stored under natural conditions in polymer pipettes for 2 years.

### Example 38 (pH > 6)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl) octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. The beaker is wrapped with nontransparent foil. 10 g of proroxan, 100 g of melatonin and 10 g of megestrol acetate are added to the obtained solution. The mass was being agitated when heated to 40°C till complete dissolution of the components. With the control by an ion meter with input impedance of > 2 GOhm pH value was 8.51. When cooled to the room temperature, the solution becomes cloudy. 980 g of transdermal composition with the following parameters are obtained:
1. Description - moving liquid cloudy yellow mass;
2. Density - 1.11 g/cm³;
3. pH 8.54;
4. Content of megestrol acetate is 11.20 mg/cm³;
5. Content of melatonin is 111.81 mg/cm³;
6. Content of proroxan is 11.23 mg/cm³;
7. Viscosity - 4.6 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.12 g/cm³;
3. pH 8.50;
4. Content of megestrol acetate is 4.31 mg/cm³;
5. Content of melatonin is 110.74 mg/cm³;
6. Content of proroxan is 5.18 mg/cm³;
7. Viscosity - 4.6 cPs.

Thus, if the pH exceeds 6, the drug is not stable when stored under natural conditions in polymer pipettes for 2 years.

### Example 39 (pH < 4)

394 g of 1,2,3-triacetoxypropane, 150 g of N,N-diethyltoluamid, 200 g of 1,2-isopropylidene glycerol, 100 g of polyoxyethylated α-tocopherol, 10 g of polyoxyethylated lanolin, 5 g of D-panthenol, 5 g of N-methyl-N-(1,2*R*,3*R*,4*R*,5*S*-pentahydroxyhexyl)-octadecanamide and 1 g of 3-(octadecyloxy)-1,2-propanediol are put into a 2L beaker fitted with an agitator. The mass is being agitated when heated to 60°C till complete dissolution of the components. It is being cooled to the room temperature when agitated. pH value is adjusted to 3.13 by adding trichloroacetic acid with the control by an ion meter with input impedance of > 2 GOhm. The beaker is wrapped with nontransparent foil. 5 g of butyroxan, 5 g of proroxan, 100 g of melatonin and 10 g of megestrol acetate are added to the obtained solution. The mass is being agitated when heated to 40°C till complete dissolution of the components. pH value is adjusted by adding trichloroacetic acid to the value of 3.02 with the control by an ion meter with input impedance of > 2 GOhm. The aggregate quantity of trichloroacetic acid used for pH level adjustment was 5 g. When cooled to the room temperature, the solution becomes cloudy. 989 g of transdermal composition with the following parameters are obtained:
1. Description - moving liquid cloudy yellow mass;
2. Density - 1.15 g/cm³;
3. pH 3.08;
4. Content of megestrol acetate is 11.50 mg/cm³;
5. Content of melatonin is 114.31 mg/cm³;
6. Content of proroxan is 11.39 mg/cm³;
7. Viscosity - 4.6 cPs.

The drug is packed in polypropylene-polyvinyl chloride pipettes of 1 ml. After 2 years of storage the parameters of the drug are as follows:
1. Description - moving cloudy liquid light-yellow mass;
2. Density - 1.12 g/cm³;
3. pH 3.10;
4. Content of megestrol acetate is 11.41 mg/cm³;
5. Content of melatonin is 114.21 mg/cm³;
6. Content of proroxan is 11.25 mg/cm³;
7. Viscosity - 4.6 cPs.

Thus, if the pH is below 4, the drug is stable when stored under natural conditions in polymer pipettes for 2 years.

When studies were conducted in animals a pronounced persistent reddening of the skin covering in the place of application of the drug which led to formation of a 0.3 mm thick skin fold that did not disappear after 20 minutes; the animals showed anxiety and tried to scrape the drug off.

## Claims

1. A method for controlling sexual behaviour and contraception in female animals, involving administering of melatonin and gestagen, **characterized in that** α-adrenergic blocking agent tropic to the posterior nuclei of the hypothalamus is further administered, while the specified BAS combination is administered in therapeutic doses once during any period of estrous cycle.

2. The method according to claim 1, **characterized in that** proroxan or(and) butyroxan are used as α-adrenergic blocking agent tropic to the nuclei of the hypothalamus.

3. A pharmaceutical composition for controlling sexual behaviour in female animals, meant for "Spot-on" transdermal administration, having pH in the range of 4.0 to 6.0 and containing therapeutic quantities of melatonin, gestagen and α-adrenergic blocking agent tropic to the posterior nuclei of the hypothalamus with the following ratio of the components (mass%):
| | |
|---|---|
| Gestagen | Therapeutic quantities |
| Melatonin | Therapeutic quantities |
| α-Adrenergic blocking agent tropic to the posterior nuclei of the hypothalamus | Therapeutic quantities |
| N-Methyl-N-(1,2*R*,3*R*,4*R*,5*S*-pentahy droxyhexyl)-octadecanamide | 0.5 |
| *D*-Panthenol | 0.5 |
| Polyoxyethylated α-tocopherol | 10 |
| Polyoxyethylated lanolin | 1 |
| 3-(Octadecyloxy)-1,2-propanediol | 0.1 |
| Carboxylic acid | 0.1-1.1 |
| N,N-Diethyltoluamid | 15 |
| 1,2-Isopropylidene glycerol | 20 |
| 1,2,3-Triacetoxypropane | Up to 100 |

4. The pharmaceutical composition according to claim 3, **characterized in that** proroxan or(and) butyroxan are used as α-adrenergic blocking agent tropic to the nuclei of the hypothalamus.

5. The pharmaceutical composition according to claim 3, **characterized in that** megestrol acetate, or (and) progesterone, or (and) oxyprogesterone capronate, or (and) dydrogesterone, or (and) pregninum, or (and) norethisterone, or (and) levonorgestrel, or (and) allylestrenol, or (and) lynestrenol, or (and) gestodene, or (and) dienogest, or (and) drospirenone, or (and) gestonorone capronate, or (and) medroxyprogesterone, or (and) mepregenol acetate, or (and) mepregenol acetate butyrate, or (and) mepregenol acetate hemisuccinate are used as gestagen.
